**Europäisches Patentamt**

**European Patent Office**

**Office européen des brevets**

(11) Veröffentlichungsnummer: **0 417 723 B1**

# (12) EUROPÄISCHE PATENTSCHRIFT

(45) Veröffentlichungstag der Patentschrift: **12.04.95**

(21) Anmeldenummer: **90117465.6**

(22) Anmeldetag: **11.09.90**

(51) Int. Cl.6: **C07C 45/35**, C07C 47/22, B01J 27/192, B01J 35/02, B01J 35/10

(54) **Verfahren zur Herstellung von Acrolein durch katalytische Gasphasenoxidation von Propen.**

(30) Priorität: **13.09.89 DE 3930534**

(43) Veröffentlichungstag der Anmeldung:
**20.03.91 Patentblatt 91/12**

(45) Bekanntmachung des Hinweises auf die
Patenterteilung:
**12.04.95 Patentblatt 95/15**

(84) Benannte Vertragsstaaten:
**AT BE CH DE DK ES FR GB GR IT LI LU NL SE**

(56) Entgegenhaltungen:
**EP-A- 0 102 641**
**EP-A- 0 279 374**
**FR-A- 2 435 456**
**US-A- 3 761 424**

(73) Patentinhaber: **Degussa Aktiengesellschaft**
**Weissfrauenstrasse 9**
**D-60311 Frankturt (DE)**

(72) Erfinder: **Böck, Wolfgang, Dr.**
**Am Häuser Graben 2**
**D-6456 Langenselbold (DE)**
Erfinder: **Arntz, Dietrich, Dr.**
**Lorsbachstrasse 32**
**D-6370 Oberursel (DE)**
Erfinder: **Prescher, Günter, Dr.**
**10 Sherwood Drive**
**Larchmont,**
**N.Y. 10538-2626 (US)**
Erfinder: **Burkhardt, Werner**
**Reichenbachstrasse 9**
**D-6486 Brachttal 2 (DE)**

**Beschreibung**

Die Erfindung betrifft ein Verfahren zur Herstellung von Acrolein durch katalytische Gasphasenoxidation von Propen mit Luft in Gegenwart von Wasserdampf und Inertgas oder vorzugsweise Abgas aus der Reaktion, aus dem die kondensierbaren Bestandteile abgetrennt sind, bei erhöhter Temperatur und einem Verhältnis zwischen Propen, Luft, Inertgas bzw. Abgas und Wasser von 1 : 6 - 9 : 3 - 12 : 0 - 5.

Die stark exotherme Umsetzung von Propen an heterogenen Katalysatoren mit einem Sauerstoff enthaltenden Gas führt neben dem erwünschten Produkt Acrolein zu einer Reihe von unerwünschten Nebenprodukten. Es ist bekannt, daß durch eine gute Abführung der Reaktionswärme, beispielsweise in Rohrbündelreaktoren, lokale Überhitzungen des Katalysators und damit einhergehende erhöhte Nebenproduktbildung vermieden werden kann.

Es ist weiterhin bekannt, daß durch Größe und äußere Form der Katalysatorkörper der Druckverlust einer Katalysatorschüttung beeinflußt werden kann. Die innere Struktur der Katalysatorkörper (Porosität, Länge der Diffusionswege) bestimmt entscheidend den Stoff- und Wärmetransport im Katalysator und hat damit neben der Zusammensetzung der katalytisch aktiven Masse maßgeblichen Einfluß auf die Selektivität.

Hohe Druckfestigkeit und Abriebfestigkeit ist eine Vorbedingung, um einen Katalysator in der industriellen Praxis einsetzen zu können. Ein hoher Abrieb würde dazu führen, daß beim Füllen der Rohre eines Rohrbündelreaktors die Streuung der Druckverluste der Einzelrohre hoch ist, woraus eine unterschiedliche Durchströmung derselben mit Beeinträchtigung der Selektivität resultiert.

In der DE-PS 31 25 061 wird ein Verfahren zur Herstellung von Acrolein unter Einsatz von Schalenkatalysatoren beschrieben.

Bei Schalenkatalysatoren werden durch die temperaturausgleichende Wirkung des inerten Trägers lokale Überhitzungen vermieden; in der relativ dünnen Schale sind die Diffusionswege für die gasförmigen Reaktanden kurz.

In der DE-OS 33 38 380 werden für die Oxidation von Propen zu Acrolein ring- bzw. hohlzylinderförmige Katalysatoren beschrieben, die aus einer Mo, Fe, Bi und W enthaltenden Masse gefertigt werden. Diese Katalysatoren kann man sich aus dem Schalenkatalysatoren abgeleitet vorstellen, indem der inerte Kern der Schalenkatalysatoren durch einen "inerten Hohlraum" ersetzt wird und die Schale in zwei gegenüberliegenden Stellen offen für den Zutritt der Reaktanden zum Hohlraum ist. Gegenüber den Schalenkatalysatoren weisen diese ring- bzw. hohlzylinderartigen Katalysatoren ein vergrößertes Verhältnis von äußerer Oberfläche zu Volumen auf. Die aktive Masse ist dadurch für die Reaktanden besser zugänglich.

Der niedrige Druckverlust und die hohe Wärmeabfuhr der Schalenkatalysatoren liegen auch hier vor. Um bei den "Hohlkatalysatoren" eine genügende mechanische Festigkeit zu erzielen, wird die aktive Masse stark verdichtet, was dazu führt, daß die innere Struktur nachteilig beeinflußt wird.

Katalysatoren in Ringform, hier allerdings mit abgerundeten Stirnflächen zwecks Verbesserung der Verfüllbarkeit, werden auch in der EP-OS 0 184 790 beschrieben, wobei jedoch weder die Katalysatormasse noch ein spezielles Herstellverfahren angegeben sind, insbesondere sind keine Maßnahmen zur Erzielung einer speziellen günstigen inneren Struktur dargestellt.

Um die aktive Masse optimal nutzen zu können, muß die innere Struktur des Katalysators so aufgebaut sein, daß eine an sich mögliche hohe Reaktionsgeschwindigkeit nicht durch eine Behinderung des Stofftransports im Inneren des Katalysators begrenzt wird.

Ein in dieser Richtung gehender Versuch ist der EP-OS 0 279 374 gezeigt. Hier ist ein Herstellungsverfahren für einen Mo, Fe, Bi enthaltenden, durch spezifische Oberfläche, Porenvolumen und Porenverteilung charakterisierten Katalysator, beschrieben.

Verfahrensbedingt können jedoch nur Katalysatorpartikel von annähernd kugelförmiger Form, d. h. mit sehr kleinem Verhältnis von Oberfläche zu Volumen erhalten werden, oder die Partikel müßten sehr klein werden. Dem sind aber für die industrielle Anwendung wegen des damit verbundenen hohen Druckverlustes Grenzen gesetzt.

Die nach bekanntem Stand der Technik hergestellten und verwendeten Katalysatoren haben bezüglich der dargelegten Gesichtspunkte einige Nachteile. Durch Einsatz verschieden geformter Körper wird entweder versucht die Diffusionswege zu verkürzen, lokale Überhitzungen zu vermeiden oder durch eine geeignete innere Struktur des Katalysators eine verbesserte Ausnutzung der Katalysatorvolumina zu erreichen. Einzelmaßnahmen dieser Art haben bisher dazu geführt, daß sich mit solchen Katalysatoren im technischen Einsatz nur eine vergleichsweise unbefriedigende Produktivität bei der Acroleingewinnung pro eingesetztem Katalysatorvolumen erreichen läßt. Dies ist wirtschaftlich von erheblichem Nachteil, da zum Ausgleich große und kostspielige Reaktoren mit hohem Füllvolumen für die Katalysatoren zur Durchführung der Reaktion eingesetzt werden müssen.

2

Der Erfindung liegt die Aufgabe zugrunde, ein verbessertes Verfahren zur Herstellung von Acrolein durch katalytische Gasphasenoxidation von Propen mit Luft zu schaffen, welches in an sich bekannter Weise in Gegenwart von Wasserdampf und Inertgas oder vorzugsweise Abgas aus der Reaktion, aus dem die kondensierbaren Bestandteile abgetrennt sind, arbeitet, höhere Temperaturen verwendet und ein Verhältnis zwischen Propen, Luft, Inertgas bzw. Abgas und Wasser von 1 : 6 - 9 : 3 - 12 : 0 - 5 vorsieht.

Es wurde gefunden, daß man mit einem Katalysator, welcher eine optimierte chemische Zusammensetzung aufweist und durch ein neues Herstellungsverfahren eine besonders günstige äußere Form und innere Struktur bekommen hat, bei Anwendung eines speziellen Temperaturbereichs und einer Prozeßführung unter leichtem Überdruck bei sehr hohen spezifischen Belastungen arbeiten kann, ohne daß dadurch ein Verlust an Selektivität auftritt. Der Katalysator und seine Gewinnung sind in der gleichlaufend eingereichten deutschen Patentanmeldung P 39 30 533.3 (89 177 KY) beansprucht.

Das erfindungsgemäße Verfahren ist im einzelnen dadurch gekennzeichnet, daß man die gasförmigen Einsatzstoffe bei 300 - 380° C und einem Absolutdruck von 1,4 - 2,2 bar über eine Katalysatormasse leitet, enthaltend die Zusammensetzung $Mo_{12}Fe_{0,4-4,0}Co_{0,4-4,0}Ni_{1,0-9,0}Bi_{0,2-2,0}$ $P(As)_{0,2-2,0}K(Rb,Cs)_{0-0,1}$ $Sm_{0,01-0,2}Si_{5-40}O_x$, wobei das Element Silicium als pyrogene oder hochdisperse gefällte Kieselsäure, Kieselsäuresol, feinteiliges Aluminiumsilikat, insbesondere in Form von Montmorillonit vorliegt und wobei die Katalysatormasse in Form einer Einzelkörperschüttung eine Kombination der Eigenschaften

a) Katalysatorkörper von beliebiger geometrischer Form, dessen Verhältnis von äußerer Oberfläche Op zu Volumen Vp oberhalb 1,6 mm$^{-1}$ liegt und dessen räumliche Ausdehnung, beschrieben durch den Durchmesser einer ihn gerade noch umschließenden Kugel, kleiner als 7,5 mm ist;

b) Porosität des Katalysatorkörpers von mindestens 0,46,
Abwesenheit von Mikroporen (< 2 nm),
Mesoporenvolumen (2 - 30 nm) von mindestens 0,03 cm$^3$/g,
sowie Makroporenvolumen (> 30 nm) von mindestens 0,30 cm$^3$/g;

c) Quecksilberdichte des Katalysatorkörpers von mindestens
1,25 g/cm$^3$;

d) spezifische Oberfläche nach BET von mindestens 10 m$^2$/g;

e) Bruchfestigkeit von mindestens 6,0 N;

f) Abrieb von unter 50 mg/g Katalysator;

g) Druckverlust von unter 1600 Pa/m einer in einem Rohr mit 2 cm Durchmesser eingebrachten Katalysatorschüttung

aufweist und wobei eine spezifische Belastung von 2 - 8 mol Propen/dm$^3$ Katalysatorschüttung/h eingestellt wird.

Der im erfindungsgemäßen Verfahren einzusetzende Katalysator weist in allen für die Gasphasenoxidation von Propen vorgeschlagenen Mischoxidformulierungen große Vorteile hinsichtlich der erzielbaren Raumzeitausbeute auf. Die für eine hohe Raumzeitausbeute erforderliche Aktivität wird durch das günstige Verhältnis zwischen äußerer Oberfläche und Volumen und die günstige innere Struktur ermöglicht. Durch das vergrößerte Verhältnis von Oberfläche zu Volumen ist die jeweilige katalytisch aktive Masse für die Reaktanden gut zugänglich und die Diffusionswege sind kurz. Die günstige innere Struktur hat zur Folge, daß der Diffusionswiderstand im Katalysatorinneren gering ist. Die durch die große innere Oberfläche bedingte hohe Aktivität kann dadurch gut ausgenutzt werden. Grundsätzlich hat der herabgesetzte Diffusionswiderstand im Katalysatorkörper auch einen günstigen Einfluß auf die Selektivität.

Der Körper des erfindungsgemäßen Katalysators kann beliebige geometrische Formen haben. Beispiel für bevorzugte Körperformen sind in der anliegenden Zeichnung gezeigt.

Eine für das Verfahren bevorzugte Variante der Katalysatorformulierung besteht aus einer Masse, enthaltend die Zusammensetzung $Mo_{12}Fe_{0,6-2,0}Co_{0,6-2,0}Ni_{2,0-6,0}Bi_{0,5-1,5}P(As)_{0,5-1,5}$ $K(Rb,Cs)_{0,001-0,05}Sm_{0,02-0,1}Si_{10-30}O_x$, wobei das Element Silicium in Form von pyrogenem $SiO_2$ und Montmorillonit im Gewichtsverhältnis 1 : 0,5 bis 1 : 4 vorliegt. Dabei hat es sich als zweckmäßig erwiesen, daß der Montmorillonit eine durch Glühbehandlung herabgesetzte spezifische Oberfläche nach BET unterhalb von 2,0 m$^2$/g hat.

Besonders vorteilhaft führt man die Umsetzung in Rohrbündelreaktoren durch, deren Rohre einen Innendurchmesser von 16 - 25 mm aufweisen.

Die im Verfahren gemäß Erfindung anzuwendenden Katalysatoren sind erhältlich, indem man

a) eine in an sich bekannter Weise durch Vereinigen von Salzlösungen der katalytisch aktiven Elemente (außer Si) gewonnenen Suspension des Kopräzipitats mit dem unlöslichen siliciumhaltigen Feststoff mischt und die dabei erhaltene Suspension unter Bedingungen sprühtrocknet, welche eine Anfangstemperatur der Trocknungsluft von 300° C - 600° C und eine Temperatur bei der Abscheidung des getrockneten Pulvers von 120 - 220° C sowie eine ein Sprühkorn mit einem mittleren Korndurchmesser

kleiner 30 µm erzeugende Zerstäubungsintensität vorsehen, wobei die Verweilzeit des Sprühkorns im Trockner 2 bis 25 sec. beträgt,

b) das trockene Sprühkorn in einem Ofen, vorzugsweise einem Drehrohrofen, bei Verweilzeiten von 5 bis 60 min und einer Spitzentemperatur im Sprühkorn von 320 bis 480° C kalziniert.

c) das kalzinierte Sprühkorn mit 5 bis 40 Gew.%, bezogen auf die Menge des Sprühkorns, eines bei einer Temperatur unter 400° C vollständig zersetzlichen Porenbildners und mit einer eine extrusionsfähige Masse ergebenden Menge an Befeuchtungs-, Gleit- und Bindemittel, deren Anteil insgesamt nicht mehr als 40 Gew.% der Sprühkornmenge ausmacht, bei einer Temperatur unterhalb 80° C in der gewünschten geometrischen Form bei einem Druck unterhalb 50 bar extrudiert und den extrudierten Strang durch Schneiden auf die Länge des gewünschten Körpers unterteilt,

d) die extrudierten Einzelkörper trocknet und dann die in einen Ofen, vorzugsweise in einen Drehrohrofen, eingebrachte zersetzbare Substanz vorsichtig ausbrennt und sie bei einer Verweilzeit von 5 bis 60 min und einer Spitzentemperatur in der Schüttung der Einzelkörper von 450 bis 650° C im Luftstrom tempert.

Dieses Katalysatorherstellungsverfahren erschließt eine Vorgehensweise, die im wesentlichen aus der Kombination einer Sprühtrocknung des Ausgangsmaterials bei definierte Bedingungen, einer Zwischentemperung innerhalb einer angegebenen Temperaturspanne, einer Extrusion des kalzinierten Sprühkorns mit definierten Mengen eines Porenbildners und üblichen Verarbeitungshilfsmitteln in gewichtsmäßig begrenzter Menge unter genauen Extrusionsbedingungen und einer abschließenden Temperung an der Luft bei angehobenem Temperaturniveau besteht. Günstig ist, wenn als Porenbildner fester Pentaerythrit mit einem mittleren Korndurchmesser kleiner 40 µm eingesetzt wird. Neben Pentaerythrit können als Porenbildner auch Cellusolepulver, Harnstoff, Oxalsäure und Polyvinylalkohol eingesetzt werden.

Durch die Sprühtrocknung der Suspension des Kopräzipitats wird bei den angegebenen Bedingungen ein kugeliges Sprühkorn mit hoher innerer Porosität erhalten. Dadurch wird die für solche Katalysatoren hohe innere Oberfläche erzeugt. Durch die Zwischentemperung werden sämtliche zersetzlichen Bestandteile aus dem Primärkorn ausgetrieben, damit bei der abschließenden Temperung keine Verringerung der Festigkeit auftreten kann.

Durch den beim Extrusionsprozeß, vorzugsweise im Korngrößenbereich des Primärkorns zugefügten Porenbildner, entsteht bei der Endtemperung ein Makroporensystem, durch das die hochaktiven mesoporösen Primärpartikel für die Reaktanden gut zugänglich werden.

Beim Extrudieren können mit Vorteil als Gleitmittel Petroleum oder Wasser und als Befeuchtungs- und Bindemittel bzw. Gleitmittel eine 1 - 10 Gew.%ige wäßrige Methylcelluloselösung, vorzugsweise in Form einer Öl- in -Wasser-Emulsion, oder als Bindemittel trockenes Methylcellulosepulver eingesetzt werden.

Die abschließende Temperung bei 450 - 650° C schließt einen vorausgehenden vorsichtigen Ausbrennvorgang während des Aufheizens ein. Das Ausbrennen und Tempern der extrudierten Einzelkörper kann aber auch in gesonderten Schritten vorgenommen werden. In beiden Fällen werden besonders gute Ergebnisse erhalten, wenn man die extrudierten Einzelkörper und den Luftstrom beim Ausbrennvorgang im Gleichstrom führt und das Ausbrennen bei höchstens 400° C vornimmt.

Bei Einsatz der genannten Katalysatoren für die Oxidation von Propen zu Acrolein mit Luft müssen die beanspruchten, sehr günstigen Betriebsbedingungen, welche zu einer hohen Produktivität führen, eingesetzt werden.

Die Erfindung wird nachfolgend durch Ausführungsbeispiele in Verbindung mit der Zeichnung weiter erläutert. Die einzige Figur der Zeichnung zeigt bevorzugt geometrische Körperformen für den Katalysator.

Die in den Beispielen für den Katalysator angegebenen Meßgrößen werden wie folgt bestimmt:

1) Bestimmung der Porosität:

Sie wird aus Quecksilber- und Helium-Dichte berechnet;

$$\text{Porosität} = (1 - \text{Hg-Dichte/He-Dichte}) \cdot 100 \ (\text{dimensionslos})$$

"Porosität" ist als der prozentuale Anteil des Leervolumens der Katalysatormasse an deren Gesamtvolumen definiert.

2) Bestimmung des Mesoporenvolumens:

Barrett, E.P.; Joyner, L.G.; Halenda, P.P. J. Am. Chem. Soc., 73 (1951), S. 373;

3) Bestimmung des Makroporenvolumens:

Hg-Einpreßverfahren mit Carlo-Erba Porosimeter 200 bis 1000 bar Druck;

4) Bestimmung der Schüttdichte, Quecksilberdichte (Scheindickte) und Heliumdichte (wahre Dichte);

Die Schüttdichte wird bestimmt, indem ein gerades Stahlrohr von 20 mm Innendurchmesser innerhalb 1 min. gleichmäßig mit 200 g Katalysator befüllt wird und die Höhe der resultierenden Katalysatorschüttung gemessen wird.

Die Quecksilberdichte wird bestimmt, indem in ein Pyknometer von 25 ml Volumen 2 g des auf 200 µm zerkleinerten Katalysators eingefüllt werden und dann das Pyknometer mit Quecksilber sorgfältig gefüllt wird. Aus den Massen des Quecksilbers, die zur Füllung des Pyknometers mit bzw. ohne Katalysastorprobe benötigt werden und der Masse der Katalysatorprobe selbst, wird die Quecksilberdichte (oder Scheindickte) des Katalysators erhalten. Die Heliumdichte (oder wahre Dichte) der Katalysatormasse wird mit einem Beckmann-Luftvergleichspyknometer bestimmt.

5) Bestimmung der BET-Oberfläche nach DIN 66131 (Meßgas $N_2$):

Ausheizbedingungen:     15 h bei 100° C getrocknet,

1 h bei 200° C im Vakuum entgast;

6) Bestimmung der Bruckfestigkeit:

Messung senkrecht zur Extrusionsrichtung mit Erweka TBA 28, arithmetisches Mittel aus 100 Einzelmessungen ± Standardabweichung;

7) Bestimmung des Abriebs:

Messung mit Roche Friabilator TA3-R, Einwaage 50 g, 10 Upm, Belastungsdauer 5 min; Ergebnisangabe als Abrieb von Katalysatorteilchen < 1 mm in mg/g Katalysator;

8) Bestimmung des Druckverlusts:

In ein Rohr von 2 cm Innendurchmesser, welches am unteren Ende mit einem Drahtnetz verschlossen ist, wird gleichmäßig innerhalb einer Minute so viel Katalysator eingefüllt, daß die Höhe der Katalysatorschüttung 1 m beträgt. Diese Schüttung wird mit 1 $Nm^3$/h Luft von 20° C durchströmt und der Druckverlust gemessen;

9) Bestimmung der Korngrößenverteilung:

Sie erfolgt mit CILAS Granulometer 715. Als Suspensionsflüssigkeit wird Äthanol eingesetzt. Zur Zerstörung von Agglomeraten wird 1 min. mit Ultraschall desagglomeriert.

10) Bestimmung der katalytischen Eigenschaften:

Die katalytische Wirkung der fertigen Katalysatoren wird in einem technischen Reaktorrohr von 20,5 mm Innendurchmesser, das von außen durch ein Salzbad gekühlt ist, bei einer Katalysatorschüttungslänge von 250 cm anhand der Umsetzung von Propen zu Acrolein getestet. Die Reaktion wird bei einer Einspeisung von 5,8 mol Propen/h, 43,5 mol Luft/h, 34,8 mol Abgas (Zusammensetzung: 5 % $O_2$, 1 % Propen, 94 % Inertgas ($CO_2$, CO, $N_2$, Ar, Propan)) und 2,9 mol $H_2O$/h und einem Druck von 1,8 bar am Eintritt in die Katalysatorschüttung durchgeführt. Die eingestellte Salzbadtemperatur, die maximal erhaltene Übertemperatur (Exotherme) in der Rohrmitte und die gemessenen Umsätze und Ausbeuten sind in Tab. 3 zusammengestellt.

Dabei ist definiert

$$\text{die Acroleinausbeute (\%) als} \quad \frac{\text{mol/h Acrolein gebildet}}{\text{mol/h Propen zugeführt}} \cdot 100$$

$$\text{die Acrylsäureausbeute (\%) als} \quad \frac{\text{mol/h Acrylsäure gebildet}}{\text{mol/h Propen zugeführt}} \cdot 100$$

der Umsatz von Propen % als

$$(1 - \frac{\text{mol/h aus dem Reaktionsrohr austretendes Propen}}{\text{mol/h in das Reaktionsrohr eingespeistes Propen}}) \cdot 100$$

die Acroleinselektivität (%) als

$$\frac{\text{Acroleinausbeute}}{\text{Propenumsatz}} \cdot 100$$

die Acrylsäureselektivität (%) als

$$\frac{\text{Acrylsäureausbeute}}{\text{Propenumsatz}} \cdot 100 \quad \text{und}$$

die Acroleinproduktivität als

$$\frac{\text{g/h Acrolein gebildet}}{\text{dm}^3 \text{ Katalysatorschüttung}}$$

Die folgenden Beispiele betreffen die Herstellung und Anwendung der in Tabelle 1 aufgeführten Katalysatoren.

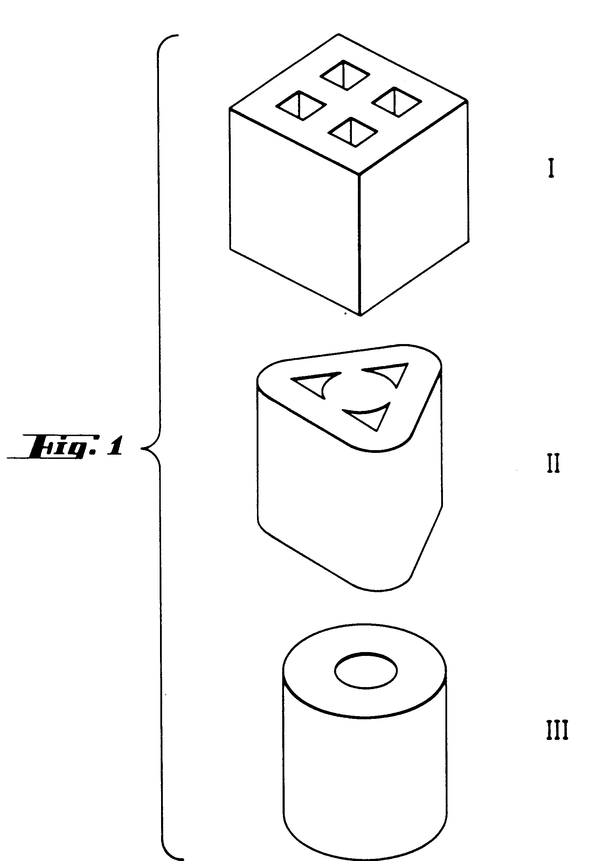

Die in Tabelle 1 unter Beispiel-Nr. 1 angegebene Katalysatorzusammensetzung wurde in der geometrischen Form III von Fig. 1 wie folgt hergestellt:

Beispiel 1

Das Kopräzipitat für die Herstellung der aktiven Katalysatorphase wird erhalten, indem man 484,8 g Fe-$(NO_3)_3 \cdot 9H_2O$, 291,0 g $Co(NO_3)_2 \cdot 6H_2O$, 1163,2 g $Ni(NO_3)_2 \cdot 6H_2O$ und 2,5 g $KNO_3$ in 3,1 l Wasser löst und unter Rühren bei 90° C zunächst eine Lösung von 17,4 g $Sm_2O_3$ in 106 g konz. $HNO_3$ zugibt. Zu dieser Lösung werden unter weiterem Rühren 601 g hochdisperse Kieselsäure (Aerosil 200) und 1202 g getemperter Montmorillonit (spez. Oberfläche nach BET < 1 m$^2$/g) gegeben.

In einem getrennten Gefäß wird bei 60° C eine Lösung von 2118,6 g $(NH_4)_6Mo_7O_{24} \cdot 6H_2O$ in 2,7 l $H_2O$ bereitet und zu dieser unter intensivem Rühren 92,2 g 85 %ige $H_3PO_4$ gegeben.

Danach werden die beiden Lösungen unter intensivem Rühren vereinigt und eine Lösung von 242,5 g $Bi(NO_3)_3 \cdot 5H_2O$ in 204 g 8,2 %iger $HNO_3$ zugegeben. Die erhaltene Suspension wird in einem Sprühtrockner bei einer Eintrittstemperatur der Trocknungsluft von 550° C getrocknet. Das Verhältnis von Trocknungsluftmenge zu versprühter Suspensionsmenge wird dabei so eingestellt, daß eine Austrittstemperatur von 170° C erhalten wird und die Suspensionsmenge wird so eingestellt, daß eine Verweilzeit von 6 sec des Sprühkorns im Sprühtrockner erhalten wird. Die Zerstäubungsintensität ist so gewählt, daß ein Sprühkorn mit einem mittleren Durchmesser $\overline{d}p$ = 25 $\mu$m erhalten wird. Das getrocknete Sprühkorn wird in einem Drehrohrofen bei einer Verweilzeit von 30 min. und einer maximalen Temperatur im Sprühkorn von 420° C kalziniert.

1,6 kg des kalzinierten Sprühkorns werden in einer Kneter/Extruder-Kombination vorgelegt und mit 0,4 kg Pentaerythrit ("feinstgemahlen") eines mittleren Korndurchmessers von $\overline{d}p$ = 40 $\mu$m 5 min. gemischt. In dieser Mischung werden 493 g einer 6 Gew.-%igen Tyloselösung gegeben, in der zuvor 26,6 g Petroleum emulgiert wurden. Diese Masse wird nun solange geknetet, bis ein homogener plastischer Zustand erreicht ist. Die Extrusion erfolgt dann bei einem Druck von 10 bar und einer Temperatur von 30° C in der extrudierten Masse.

Der extrudierte Hohlstrang wird nach jeweils 5 mm abgeschnitten, die abgeschnittenen Ringe von 5 mm Durchmesser, 5 mm Länge und 1,8 mm Innendurchmesser vorsichtig bei 80° C getrocknet.

Die getrockneten Ringe werden in ein beheiztes Drehrohr eingetragen und bei einer Drehzahl von 2 min$^{-1}$ und Luftüberschuß auf eine Spitzentemperatur von 600° C in der durch das Drehrohr wandernden Schüttung erhitzt. Die Verweilzeit im Drehrohr beträgt dabei 30 min.

Die an der Katalysatorzusammensetzung bzw. deren Vorstufen gemessenen physikalischen Eigenschaften sind in Tabelle 2 und die Katalysatorwirkung bei der Herstellung von Acrolein in der Tabelle 3 zusammengefaßt.

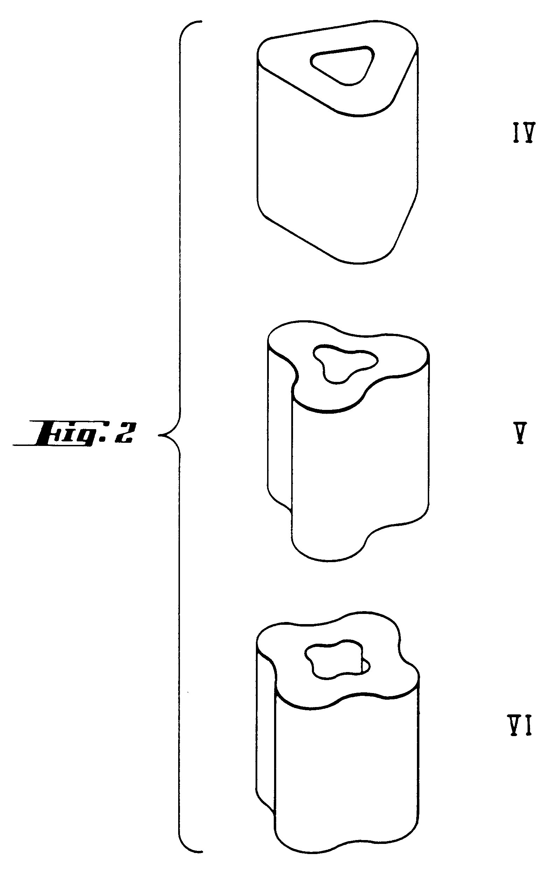

In Beispiel 2 wurde die gleiche Katalysatorzusammensetzung wie in Beispiel 1 in der geometrischen Form VI von Fig. 2 hergestellt.

Beispiel 2

Entsprechend Beispiel 1 wird das Kopräzipitat der aktiven Katalysatorphase hergestellt, getrocknet, kalziniert und in einer Kneter/Extruder-Kombination solange geknetet, bis ein homogener plastischer Zustand erreicht ist. Die Extrusion erfolgt bei einem Druck von 15 bar und einer Temperatur von 32°C in der extrudierten Masse. Der in Form VI von Fig. 2 extrudierten Hohlstrang wird nach jeweils 5 mm abgeschnitten, die abgeschnittenen Katalysatorrohlinge getrocknet und wie in Beispiel 1 im Drehrohr getempert.

In Beispiel 3 wurde die gleiche Katalysatorzusammensetzung wie in Beispiel 1 in der geometrischen Form II von Fig. 1 hergestellt.

Beispiel 3

Entsprechend Beispiel 1 wird das Kopräzipitat der aktiven Katalysatorphase hergestellt, getrocknet, kalziniert und in einer Kneter/Extruder-Kombination solange geknetet, bis ein homogener plastischer Zustand erreicht ist. Die Extrusion erfolgt bei einem Druck von 15 bar und einer Temperatur von 32°C in der extrudierten Masse. Der in Form II von Fig. 1 extrudierte Hohlstrang wird nach jeweils 5 mm abgeschnitten, die abgeschnittenen Katalysatorrohlinge getrocknet und wie in Beispiel 1 im Drehrohr getempert.

Im Vergleichsbeispiel 1 wurde die gleiche Katalysatorzusammensetzung wie in Beispiel 1 in Form von Strangpreßlingen extrudiert.

Vergleichsbeispiel 1

Entsprechend Beispiel 1 wird das Kopräzipitat der aktiven Katalysatorphase hergestellt, getrocknet, kalziniert und in einer Kneter/Extruder-Kombination solange geknetet, bis ein homogener plastischer Zustand erreicht ist. Die Extrusion erfolgt bei einem Druck von 8 bar und einer Temperatur von 25°C in der extrudierten Masse. Die in Form eines Vollstranges von 5 mm Durchmesser extrudierte Katalysatormasse wird nach jeweils 5 mm abgeschnitten, die abgeschnittenen Katalysatorrohlinge getrocknet und wie in Beispiel 1 im Drehrohr getempert.

In den folgenden Beispielen 4, 5 und 6 werden verschiedene Katalysatorzusammensetzungen in der geometrischen Form III von Fig. 1 wie in Beispiel 1 hergestellt.

Beispiel 4

Das Kopräzipitat für die Herstellung der aktiven Katalysatorphase wird erhalten, indem man 242,4 g Fe(NO$_3$ • 9H$_2$O, 232,8 g Co(NO$_3$)$_2$ • 6H$_2$O, 1744,8 g Ni(NO$_3$)$_2$ • 6H$_2$O und 5,1 g KNO$_3$ in 3,1 l Wasser löst und unter Rühren bei 90°C zunächst eine Lösung von 34,9 g Sm$_2$O$_3$ in 212 g konz. HNO$_3$ zugibt. In dieser Lösung werden unter weiterem Rühren 300,5 g hochdisperse Kieselsäure (Aerosil 200) und 300,5 g getemperter Montmorillonit (spez. Oberfläche nach BET < 1 m$^2$/g) gegeben. In einem getrennten Gefäß wird bei 60°C eine Lösung von 2118,6 g (NH$_4$)$_6$Mo$_7$O$_{24}$ • 6H$_2$O in 2,7 l H$_2$O bereitet und zu dieser unter intensivem Rühren 115,3 g 85 %ige H$_3$PO$_4$ gegeben. Danach werden die beiden Lösungen unter intensivem Rühren vereinigt und eine Lösung von 485,1 g Bi(NO$_3$)$_3$ • 5H$_2$O in 408 g 8,2 %iger HNO$_3$ zugegeben. Die erhaltene Suspension wird in einem Sprühtrockner bei einer Eintrittstemperatur der Trocknungsluft von 550°C getrocknet. Das Verhältnis von Trocknungsluftmenge zu versprühter Suspensionsmenge wird dabei so eingestellt, daß eine Austrittstemperatur von 170°C erhalten wird und die Suspensionsmenge wird so eingestellt, daß eine Verweilzeit von 6 sec. des Sprühkorns im Sprühtrockner erhalten wird. Die Zerstäubungsintensität ist so gewählt, daß ein Sprühkorn mit einem mittleren Durchmesser $\overline{d}p$ = 25 µm erhalten wird. Das getrocknete Sprühkorn wird in einem Drehrohrofen bei einer Verweilzeit von 30 min. und einer maximalen Temperatur im Sprühkorn von 420°C kalziniert.

Die Weiterverarbeitung des kalzinierten Sprühkorns erfolgt wie in Beispiel 1, außer daß der extrudierte Hohlstrang einen Innendurchmesser von 2 mm hat.

Beispiel 5

Das Kopräzipitat für die Herstellung der aktiven Katalysatorphase wird erhalten, indem man 808,0 g Fe-$(NO_3)_3$ • $9H_2O$, 320,1 g $Co(NO_3)_2$ • $6H_2O$, 1744,8 g $Ni(NO_3)_2$ • $6H_2O$ und 0,1 g $KNO_3$ in 3,1 l Wasser löst und unter Rühren bei 90° C zunächst eine Lösung von 34,9 g $Sm_2O_3$ in 212 g konz. $HNO_3$ zugibt. In dieser Lösung werden unter weiterem Rühren 600,9 g hochdisperse Kieselsäure (Aerosil 200) und 1201,8 g getemperter Montmorillonit (spez. Oberfläche nach BET < 1 $m^2/g$) gegeben. In einem getrennten Gefäß wird bei 60° C eine Lösung von 2118,6 g $(NH_4)_6Mo_7O_{24}$ • $6H_2O$ in 2,7 l $H_2O$ bereitet und zu dieser unter intensivem Rühren 115,3 g 85 %ige $H_3PO_4$ gegeben. Danach werden die beiden Lösungen unter intensivem Rühren vereinigt und eine Lösung von 485,1 g $Bi(NO_3)_3$ • $5H_2O$ in 408 g 8,2 %iger $HNO_3$ zugegeben. Die erhaltene Suspension wird in einem Sprühtrockner bei einer Eintrittstemperatur der Trocknungsluft von 550° C getrocknet. Das Verhältnis von Trocknungsluftmenge zu versprühter Suspensionsmenge wird dabei so eingestellt, daß eine Austrittstemperatur von 170° C erhalten wird und die Suspensionsmenge wird so eingestellt, daß eine Verweilzeit von 6 sec. des Sprühkorns im Sprühtrockner erhalten wird. Die Zerstäubungsintensität ist so gewählt, daß ein Sprühkorn mit einem mittleren Durchmesser $\bar{dp}$ = 25 $\mu$m erhalten wird. Das getrocknete Sprühkorn wird in einem Drehrohrofen bei einer Verweilzeit von 30 min. und einer maximaler Temperatur im Sprühkorn von 420° C kalziniert.

Die Weiterverarbeitung des kalzinierten Sprühkorns erfolgt wie in Beispiel 4.

Beispiel 6

Das Kopräzipitat für die Herstellung der aktiven Katalysatorphase wird erhalten, indem man 646,4 g Fe-$(NO_3)_3$ • $9H_2O$, 174,6 g $Co(NO_3)_2$ • $6H_2O$, 1744,8 g $Ni(NO_3)_2$ • $6H_2O$ und 0,1 g $KNO_3$ in 3,1 l Wasser löst und unter Rühren bei 90° C zunächst eine Lösung von 34,9 g $Sm_2O_3$ in 212 g konz. $HNO_3$ zugibt. In dieser Lösung werden unter weiterem Rühren 600,9 g hochdisperse Kieselsäure (Aerosil 200) und 1201,8 g getemperter Montmorillonit (spez. Oberfläche nach BET < 1 $m^2/g$) gegeben. In einem getrennten Gefäß wird bei 60° C eine Lösung von 2118,6 g $(NH_4)_6Mo_7O_{24}$ • $6H_2O$ in 2,7 l $H_2O$ bereitet und zu dieser unter intensivem Rühren 115,3 g 85 %ige $H_3PO_4$ gegeben. Danach werden die beiden Lösungen unter intensivem Rühren vereinigt und eine Lösung von 727,7 g $Bi(NO_3)_3$ • $5H_2O$ in 612,0 g 8,2 %iger $HNO_3$ zugegeben. Die erhaltene Suspension wird in einem Sprühtrockner bei einer Eintrittstemperatur der Trocknungsluft von 550° C getrocknet. Das Verhältnis von Trocknungsluftmenge zu versprühter Suspensionsmenge wird dabei so eingestellt, daß eine Austrittstemperatur von 170° C erhalten wird und die Suspensionsmenge wird so eingestellt, daß eine Verweilzeit von 6 sec. des Sprühkorns im Sprühtrockner erhalten wird. Die Zerstäubungsintensität ist so gewählt, daß ein Sprühkorn mit einem mittleren Durchmesser $\bar{dp}$ = 25 $\mu$m erhalten wird. Das getrocknete Sprühkorn wird in einem Drehrohrofen bei einer Verweilzeit von 30 min. und einer maximalen Temperatur im Sprühkorn von 420° C kalziniert.

Die Weiterverarbeitung des kalzinierten Sprühkorns erfolgt wie in Beispiel 4.

Im Beispiel 7 wird die gleiche Katalysatorzusammensetzung wie in Beispiel 1 verformt, außer daß bei Beispiel 7 statt Pentaerythrit Cellulosepulver als Porenbildner eingesetzt wurde.

Beispiel 7

Die Herstellung des kalzinierten Sprühkorns erfolgt wie in Beispiel 1.

1,6 kg des kalzinierten Sprühkorns werden in einer Kneter/ExtruderKombination vorgelegt und mit 0,4 kg Cellulosepulver mit einem mittleren Korndurchmesser $\bar{dp}$ = 36 $\mu$m als Porenbildner 5 min. gemischt.

Die Weiterverarbeitung erfolgt wie in Beispiel 1.

Im Vergleichsbeispiel 2 wird der Katalysator wie in Beispiel 1 hergestellt, wobei aber bei der Verformung kein Porenbildner zugesetzt wird.

Vergleichsbeispiel 2

Die Herstellung des kalzinierten Sprühkorns erfolgt wie in Beispiel 1.

1,6 kg des kalzinierten Sprühkorns werden in einer Kneter/Extruder-Kombination vorgelegt. Dazu werden 394,4 g einer 6 Gew.-%igen Tyloselösung gegeben, in der zuvor 21,3 g Petroleum emulgiert wurden.

Diese Masse wird solange geknetet, bis ein homogener plastischer Zustand erreicht ist. Die Extrusion erfolgt in Form III von Figur 1 bei einem Druck von 14 bar und einer Temperatur von 32° C in der extrudierten Masse.

Die Weiterverarbeitung der extrudierten Masse erfolgt wie in Beispiel 1, wobei die Spitzentemperatur bei der Temperung im Drehrohr auf 620° C angehoben wird.

## TABELLE 1

| Beispiele | Vergl.-Beisp. | Mo | Fe | Co | Ni | Bi | P | K | Sm | Si als Montmorillonit + hochdisp.Kiesels. |
|---|---|---|---|---|---|---|---|---|---|---|
| 1,2,3,7 | 1,2 | 12 | 1,2 | 1,0 | 4,0 | 0,5 | 0,8 | 0,025 | 0,1 | 30 |
| 4 | | 12 | 0,6 | 0,8 | 6,0 | 1,0 | 1,0 | 0,05 | 0,2 | 10 |
| 5 | | 12 | 2,0 | 1,1 | 6,0 | 1,0 | 1,0 | 0,001 | 0,2 | 30 |
| 6 | | 12 | 1,6 | 0,6 | 6,0 | 1,5 | 1,0 | 0,001 | 0,2 | 30 |

Katalysatorzusammensetzung (Atomzahlen)

EP 0 417 723 B1

**TABELLE 2**  physik. chem. Katalysatorcharakterisierung

| Beisp. | V-Beisp. | $O_p/V_p$ mm$^{-1}$ | $d_K$* mm | Porosität – | $V_{meso}$ ml/g | $V_{makro}$ ml/g | Schutt- g/ml | He- g/ml | Hg-Dichte g/ml | BET-Oberfl. m$^2$/g | Bruchf. N | Abrieb mg/g | $\Delta p$ Pa/m |
|---|---|---|---|---|---|---|---|---|---|---|---|---|---|
| 1 |   | 1,65 | 7,1 | 0,51 | 0,05 | 0,38 | 0,76 | 3,48 | 1,71 | 14 | 14,9 | 16 | 1450 |
| 2 |   | 2,00 | 7,1 | 0,62 | 0,05 | 0,48 | 0,60 | 3,49 | 1,30 | 16 | 9,8 | 32 | 1400 |
| 3 |   | 2,10 | 7,1 | 0,62 | 0,04 | 0,42 | 0,70 | 2,54 | 1,30 | 16 | 15,4 | 48 | 1340 |
|   | 1 | 1,2 | 7,1 | 0,64 | 0,04 | 0,42 | 0,79 | 3,59 | 1,30 | 16 | 18,9 | 20 | 1520 |
| 4 |   | 1,73 | 7,1 | 0,56 | 0,03 | 0,39 | 0,70 | 4,02 | 1,77 | 12 | 6,2 | 8 | 1260 |
| 5 |   | 1,73 | 7,1 | 0,56 | 0,04 | 0,30 | 0,72 | 4,05 | 1,79 | 11 | 10,7 | 4 | 1190 |
| 6 |   | 1,73 | 7,1 | 0,56 | 0,03 | 0,29 | 0,73 | 4,08 | 1,81 | 12 | 7,7 | 14 | 1290 |
| 7 |   | 1,65 | 7,1 | 0,62 | 0,04 | 0,33 | 0,76 | 3,51 | 1,30 | 16 | 14,4 | 18 | 1430 |
|   | 2 | 1,65 | 7,1 | 0,44 | 0,05 | 0,25 | 0,87 | 3,46 | 1,93 | 13 | 16,8 | 11 | 1400 |

* $d_K$ = Durchmesser der den Katalysator gerade noch umschließenden Kugel

TABELLE 3  Katalysatorwirkung bei der Herstellung von Acrolein

| Beisp. | Vergl.-Beisp. | Salzbadtemp. °C | Umsatz % | Exotherme grd | Acrol. Ausb. % | Acryls. Ausb. % | Acrol. Selekt. % | Acryls. Selekt. % | Produktivität g Acrol./dm³ Katal./h |
|---|---|---|---|---|---|---|---|---|---|
| 1 | | 331 | 94,2 | 81 | 81,8 | 7,0 | 86,8 | 7,4 | 322 |
| 2 | | 347 | 93,4 | 78 | 81,4 | 7,3 | 87,1 | 7,8 | 320 |
| 3 | | 345 | 92,7 | 76 | 80,9 | 7,1 | 87,2 | 7,7 | 318 |
| | 1 | 330 | 93,1 | 78 | 77,6 | 8,5 | 83,3 | 9,1 | 273* |
| 4 | | 364 | 91,1 | 72 | 80,3 | 8,7 | 88,1 | 9,5 | 316 |
| 5 | | 344 | 90,9 | 76 | 80,1 | 9,0 | 88,1 | 9,9 | 315 |
| 6 | | 359 | 91,3 | 72 | 80,3 | 8,8 | 87,9 | 9,6 | 316 |
| 7 | | 348 | 92,6 | 77 | 80,4 | 6,9 | 86,8 | 7,5 | 317 |
| | 2 | 350 | 90,0 | 74 | 77,1 | 6,0 | 85,6 | 6,7 | 272* |

* Einspeisung 5,2 Mol Propen/h

## Patentansprüche

1. Verfahren zur Herstellung von Acrolein durch katalytische Gasphasenoxidation von Propen mit Luft in Gegenwart von Wasserdampf und Inertgas oder vorzugsweise Abgas aus der Reaktion, aus dem die kondensierbaren Bestandteile abgetrennt sind, bei erhöhter Temperatur und einem Verhältnis zwischen Propen, Luft, Inertgas bzw. Abgas und Wasser von 1 : 6 - 9 : 3 - 12 : 0 - 5,
**dadurch gekennzeichnet,**
daß man die gasförmigen Einsatzstoffe bei 300 - 380° C und einem Absolutdruck von 1,4 - 2,2 bar über eine Katalysatormasse leitet, enthaltend die Zusammensetzung $Mo_{12}Fe_{0,4-4,0}Co_{0,4-4,0}Ni_{1,0-9,0}$ $Bi_{0,2-2,0}$ $P(As)_{0,2-2,0}K(Rb,Cs)_{0-0,1}$ $Sm_{0,01-0,2}$ $Si_{5-40}O_x$, wobei das Element Silicium als pyrogene oder hochdisperse gefällte Kieselsäure, Kieselsäuresol, feinteiliges Aluminiumsilikat, insbesondere in Form von Montmorillonit vorliegt und wobei die Katalysatormasse in Form einer Einzelkörperschüttung eine Kombination der Eigenschaften

a) Katalysatorkörper von beliebiger
geometrischer Form, dessen Verhältnis von äußerer Oberfläche Op zu Volumen Vp oberhalb 1,6 $mm^{-1}$ liegt und dessen räumliche Ausdehnung, beschrieben durch den Durchmesser einer ihn gerade noch umschließenden Kugel, kleiner als 7,5 mm ist;
b) Porosität des Katalysatorkörpers von mindestens 0,46;
Abwesenheit von Mikroporen (< 2 nm),
Mesoporenvolumen (2 - 30 nm) von mindestens 0,03 $cm^3$/g
sowie Makroporenvolumen (> 30 nm) von mindestens 0,30 $cm^3$/g;
c) Quecksilberdichte des Katalysatorkörpers von mindestens 1,25 $g/cm^3$;
d) spezifische Oberfläche nach BET von mindestens 10 $m^2$/g;
e) Bruchfestigkeit von mindestens 6,0 N;
f) Abrieb von unter 50 mg/g Katalysator;
g) Druckverlust von unter 1600 Pa/m einer in einem Rohr mit 2 cm Durchmesser eingebrachten Katalysatorschüttung
aufweist und wobei eine spezifische Belastung von 2 - 8 mol Propen/$dm^3$ Katalysatorschüttung/h eingestellt wird.

2. Verfahren nach Anspruch 1,
**dadurch gekennzeichnet,**
daß die Katalysatormasse die Zusammensetzung $Mo_{12}Fe_{0,6-2,0}Co_{0,6-2,0}Ni_{2,0-6,0}Bi_{0,5-1,5}P(As)_{0,5-1,5}$ K-$(Rb,Cs)_{0,001-0,05}Sm_{0,02-0,1}Si_{10-30}O_x$, wobei das Element Silicium in Form von pyrogenem $SiO_2$ und Montmorillonit im Gewichtsverhältnis 1 : 0,5 bis 1 : 4 vorliegt, enthält.

3. Verfahren nach Anspruch 1 oder 2,
**dadurch gekennzeichnet,**
daß der Montmorillonit eine durch Glühbehandlung herabgesetzte spezifische Oberfläche nach BET unterhalb von 2,0 $m^2$/g hat.

4. Verfahren nach den Ansprüchen 1 bis 3,
**dadurch gekennzeichnet,**
daß man die Umsetzung in Rohrbündelreaktoren durchführt, deren Rohre einen Innendurchmesser von 16 - 25 mm aufweisen.

**Claims**

1. A process for the production of acrolein by catalytic gas-phase oxidation of propene with air in the presence of steam and an inert gas or, preferably, waste gas from the reaction, from which the condensible constituents have been removed, at elevated temperature and in a ratio of propene to air to inert gas or waste gas to water of 1 : 6 - 9 : 3 - 12 : 0 - 5, characterized in that the gaseous starting materials are passed at 300 to 380°C under an absolute pressure of 1.4 to 2.2 bar over a catalyst mass having the composition

$Mo_{12}Fe_{0.4-4.0}Co_{0.4-4.0}Ni_{1.0-9.0}Bi_{0.2-2.0}$
$P(As)_{0.2-2.0}K(Rb,Cs)_{0-0.1}Sm_{0.01-0.2}Si_{5-40}O_x$,

the element silicon being present in the form of pyrogenic or highly disperse precipitated silica, silica sol, finely divided aluminium silicate and, more particularly, in the form of montmorillonite and the catalyst mass in the form of a bed of individual catalyst elements having the following properties in combination:

a) catalyst elements of any geometric shape in which the ratio of outer surface Op to volume Vp is above 1.6 mm$^{-1}$ and of which the spatial dimension, defined by the diameter of a sphere which still just surrounds it, is smaller than 7.5 mm;

b) a porosity of the catalyst of at least 0.46,

the absence of micropores (< 2 nm),

a mesopore volume (2 - 30 nm) of at least 0.03 cm$^3$/g and

a macropore volume (> 30 nm) of at least 0.30 cm$^3$/g;

c) a mercury density of the catalyst element of at least 1.25 g/cm$^3$;

d) a specific BET surface of at least 10 m$^2$/g;

e) a breaking strength of at least 6.0 N;

f) an abrasion of less 50 mg/g catalyst;

g) a pressure loss of less than 1,600 Pa/m of a catalyst bed introduced into a 2 cm diameter tube, and a specific load of 2 - 8 mol propene/dm$^3$ catalyst bed/h being adjusted.

2. A process as claimed in claim 1, characterized in that the catalyst mass has the following composition:

$$Mo_{12}Fe_{0.6-2.0}Co_{0.6-2.0}Ni_{2.0-6.0}Bi_{0.5-1.5}$$
$$P(As)_{0.5-1.5}K(Rb,Cs)_{0.001-0.05}Sm_{0.02-0.1}Si_{10-30}O_x,$$

the element silicon being present in the form of pyrogenic $SiO_2$ and montmorillonite in a ratio by weight of 1:0.5 to 1:4.

3. A process as claimed in claim 1 or 2, characterized in that the montmorillonite has a specific BET surface reduced by calcination of less than 2.0 m$^2$/g.

4. A process as claimed in claims 1 to 3, characterized in that the reaction is carried out in tube bundle reactors of which the tubes have an internal diameter of 16 to 25 mm.

**Revendications**

1. Procédé de préparation d'acroléine par oxydation catalytique en phase gazeuse de propène à l'air en présence de vapeur d'eau et de gaz inerte ou de préférence de gaz rejeté provenant de la réaction, à partir duquel les composants condensables sont séparés, à haute température et avec un rapport entre propène, air, gaz inerte ou gaz rejeté et eau de 1 : 6 - 9 : 3 - 12 : 0 - 5, caractérisé en ce que l'on envoie les produits de départ gazeux, à 300-380 °C et sous une pression absolue de 1,4 - 2,2 bars, sur une masse de catalyseur, ayant la composition $MO_{12}Fe_{0,4-4,0}$ $Co_{0,4-4,0}$, $Ni_{1,0-9,0}$, $Bi_{0,2-2,0}$, $P(As)_{0,2-2,0}$, $K(Rb,Cs)_{0-0,1}$ $Sm_{0,01-0,2}$, $Si_{5-40}O_x$, l'élément silicium se trouvant sous forme d'acide silicique pyrogéné ou précipité et fortement dispersé, de sol d'acide silicique, de silicate d'aluminium finement divisé, en particulier sous forme de montmorillonite et la masse de catalyseur, sous forme d'un lit en vrac de corps solides individuels présentant une combinaison des propriétés suivantes :

a) un corps de catalyseur de forme géométrique quelconque, dont le rapport de la superficie externe Op au volume Vp est supérieur à 1,6 mm$^{-1}$, et dont l'expression spatiale, définie par le diamètre d'une sphère l'entourant exactement, est inférieure à 7,5 mm ;

b) une porosité du corps de catalyseur d'au moins 0,46, absence de micropores (< 2 nm) ;

volume des mesopores (2 - 30 nm) d'au moins 0,03 cm$^3$/g, ainsi que,

volume des macropores (> 30 nm) d'au moins 0,30 cm$^3$/g ;

c) une densité au mercure du corps de catalyseur d'au moins 1,25 g/cm$^3$ ;

d) une surface spécifique selon BET d'au moins 10 m$^2$/g ;

e) une résistance à la rupture d'au moins 6,0 N ;

f) une abrasion de moins de 50 mg/g de catalyseur ;

g) une perte de charge de moins de 1600 Pa/m d'un lit de catalyseur introduit dans un tube de 2 cm de diamètre et,

une charge spécifique de 2 - 8 mol de propène/dm$^3$ de lit en vrac de catalyseur/h étant établie.

2. Procédé selon la revendication 1, caractérisé en ce que la masse de catalyseur a la composition : $Mo_{12}Fe_{0,6-2,0}Co_{0,6-2,0}$, $Ni_{2,0-6,0}Bi_{0,5-1,5}$, $P(As)_{0,5-1,5}$ $K(Rb,Cs)_{0-001-0,05}$, $Sm_{0,02-0,1}$, $Si_{10-30}O_x$, l'élément silicium se trouvant sous forme de $SiO_2$ pyrogéné et de montmorillonite en rapport pondéral 1 : 0,5 à 1 : 4.

3. Procédé selon la revendication 1 ou 2, caractérisé en ce que la montmorillonite a une surface spécifique selon BET diminuée par traitement de calcination à moins de 2,0 $m^2/g$.

4. Procédé selon les revendications 1 à 3, caractérisé en ce que l'on effectue la réaction dans des réacteurs à faisceaux de tubes, dont les tubes présentent un diamètre intérieur de 16-25 mm.

Fig. 1

I

II

III

*Fig. 2*

IV

V

VI